# EUROPEAN PATENT APPLICATION

(11) **EP 4 640 785 A1**
(43) Date of publication of application: **29.10.2025**
(21) Application number: 23906835.6
(22) Date of filing: 12.12.2023
(51) Int. Cl.: C09K 3/00, C08K 5/372, C08L 101/00

(54) **ULTRAVIOLET LIGHT ABSORBING AGENT**

(30) Priority: 23.12.2022 JP 2022206649
(71) Applicant: Miyoshi Oil & Fat Co., Ltd., Tokyo 124-0006 (JP)
(72) Inventor: KANEKO Kotaro, Tokyo 124-0006 (JP); NAKAMURA Daisuke, Tokyo 130-0012 (JP); KAWAI Koji, Tokyo 130-0012 (JP)
(74) Representative: Angerhausen, Christoph
(86) International application number: PCT/JP2023/044487
(87) International publication number: WO 2024/135470

(57) **Abstract**

Provided are an ultraviolet absorber in which a slope to the long-wavelength side of the absorption peak is steep (the absolute value of the slope is large) and which efficiently absorbs light from a long-wavelength ultraviolet light region (350 to 400 nm) to a short-wavelength visible light region (400 to 420 nm) while inhibiting the absorption of visible light (400 to 800 nm) by adjusting the added amount thereof; and a transparent member and resin composition containing said ultraviolet light absorber. The ultraviolet absorber according to the present invention is represented by a general formula (1) defined as: 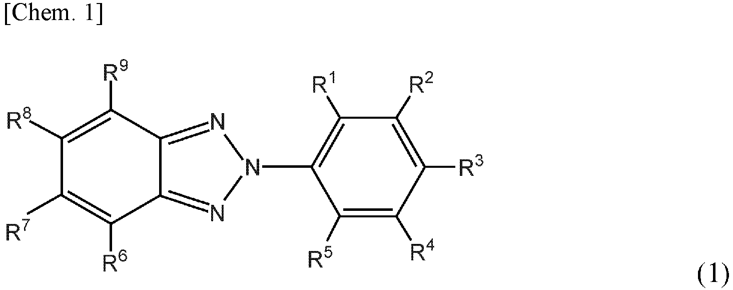 wherein, in the formula, each of R¹ and R³ to R⁹ independently represents a monovalent group selected from a hydrogen atom, a hydrocarbon-containing group, a sulfur-containing group, a nitrogen-containing group, and an oxygen-containing group. At least one of R⁶ to R⁹ represents a monovalent sulfur-containing group that has a sulfide bond. R² represents a hydrogen atom.

## Description

### Technical Field

The present invention relates to an ultraviolet absorber.

### Background Art

Various ultraviolet absorbers are used in various organic and inorganic materials for suppressing the absorption or transmission of ultraviolet rays.

In recent years, there has been a growing demand for materials that can transmit visible light at wavelengths near ultraviolet rays while efficiently absorbing ultraviolet rays at wavelengths close to visible light in the wavelength interface between ultraviolet and visible light.

The present applicant disclosed a 2-phenylbenzotriazole compound having a sulfur-containing group as an ultraviolet absorber (Patent documents 1 and 2).

### Prior Art Documents

### Patent documents

Patent document 1: WO-A-2016/021664
Patent document 2: WO-A-2019/087983

### Summary of Invention

### Technical Problem

However, in some cases, there may be wavelengths within a wavelength range of several tens of nanometers that require transmission and absorption, but conventional ultraviolet absorbers have not been able to meet this requirement because the absorption of ultraviolet rays at wavelengths close to visible light tends to broaden the base of the absorption peak into the range of visible light near ultraviolet rays, resulting in an issue of yellow coloration of the object to which the absorber is added.

To meet this requirement, the absorption peak necessitates a characteristic in which the peak has a steep slope (having a large absolute value of the slope) on the longer-wavelength side of the peak, and conventional UV absorbers have room for improvement in this respect.

The present invention has been made in view of the above circumstances; it is an object of the present invention to provide an ultraviolet absorber in which a slope to the long-wavelength side of the absorption peak is steep (the absolute value of the slope is large) and which efficiently absorbs light from a long-wavelength ultraviolet light region (350 to 400 nm) to a short-wavelength visible light region (400 to 420 nm) while inhibiting the absorption of visible light (400 to 800 nm) by adjusting the added amount thereof; and a transparent member and resin composition containing said ultraviolet light absorber.

### Solution to Problem

The inventors of the present application conducted a series of studies to address the above objectives and discovered that a 2-phenylbenzotriazole compound with a sulfur-containing group in which a hydrogen atom is bonded to one of carbon atoms meta-positioned relative to the carbon atom bonded to the nitrogen atom of the phenyl group within the 2-phenylbenzotriazole frame structure, enhances the sharpness of the light absorption peak or transmittance curve, and it allows suppression of the transmission of wavelengths intended for absorption while efficiently transmitting wavelengths intended for transmission when these wavelengths intended for absorption and transmission are in close proximity, thus completing the invention.

That is, the ultraviolet absorber according to the present invention is characterized by a general formula (1) defined as: 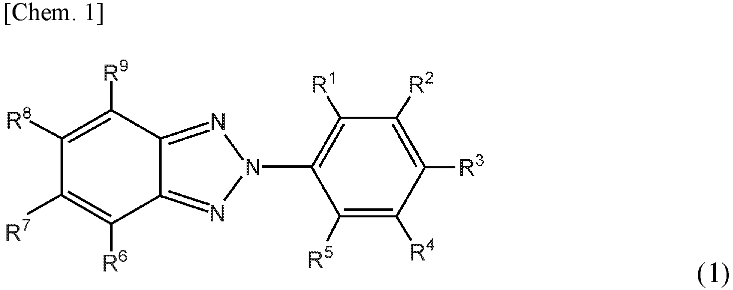 wherein, in the formula, each of R¹ and R³ to R⁹ independently represents a monovalent group selected from a hydrogen atom, a hydrocarbon-containing group, a sulfur-containing group, a nitrogen-containing group, and an oxygen-containing group. At least one of R⁶ to R⁹ represents a monovalent sulfur-containing group that has a sulfide bond. R² represents a hydrogen atom.

The transparent member according to the present invention contains the ultraviolet light absorber.

The resin composition according to the present invention contains the ultraviolet light absorber and a resin.

### Advantageous Effects of Invention

The ultraviolet absorber according to the present invention exhibits a light absorption peak having a steep slope to the long-wavelength side of the peak (the absolute value of the slope is large), and the ultraviolet absorber efficiently absorbs light from a long-wavelength ultraviolet light region (350 to 400 nm) to a short-wavelength visible light region (400 to 420 nm) while inhibiting the absorption of visible light (400 to 800 nm) by adjusting the added amount thereof.

The transparent member and resin composition according to the present invention can efficiently absorb light from a long-wavelength ultraviolet light region (350 to 400 nm) to a short-wavelength visible light region (400 to 420 nm) while inhibiting the light absorption of the visible light region (400 to 800 nm) by adjusting the added amount of the ultraviolet absorber of the present invention, thus inhibiting the yellowing thereof.

### Brief Description of Drawings

FIG. 1 illustrates UV-Vis absorption spectra of compounds 1 and 22.
FIG. 2 illustrates transmission spectra of solutions of compound 1 having concentrations of 0.075wt% and 0.75wt% and of solutions of compound 22 having concentrations of 0.025wt% and 0.25wt%.
FIG. 3 illustrates the transmission spectra of solutions of compounds 1 and 22 having concentrations of 0.4wt%.
FIG. 4 illustrates the transmission spectra of acrylic melamine resin films of compounds 7 and 23.

### Description of Embodiments

Embodiments for carrying out the present invention will be specifically described hereunder.

The numerals as used herein for indicating the number of carbon atoms are integers.

### (Ultraviolet absorber)

The ultraviolet absorber according to the present invention is represented by the formula (1).

In the formula (1), R² represents a hydrogen atom.

In the formula (1), each of R¹ and R³ to R⁹ independently represents a hydrogen atom or a monovalent group selected from a hydrocarbon-containing group, a sulfur-containing group, a nitrogen-containing group, and an oxygen-containing group. At least any one of R⁶ to R⁹ represents a monovalent sulfur-containing group that has a sulfide bond.

The hydrocarbon-containing group preferably has 1 to 22 carbon atoms. The hydrocarbon-containing group contains a hydrocarbon whose hydrogen atoms may be substituted by a sulfur-containing group, an oxygen-containing group, a nitrogen-containing group, or a halogen, or whose base end(s) thereof or carbon-carbon bonds therein may be interrupted by a sulfur-containing group, a nitrogen-containing group, or an oxygen-containing group. Hydrocarbon-containing groups containing no heteroatoms are categorized as hydrocarbon groups. Of these, preferred is a hydrocarbon group, particularly a hydrocarbon group having an oxygen-containing group.

Examples of the hydrocarbon include, but are not particularly limited to, a saturated or unsaturated aliphatic hydrocarbon, an aromatic ring-containing aliphatic hydrocarbon, an aromatic hydrocarbon, an aliphatic ring-containing aliphatic hydrocarbon, and an aliphatic cyclic hydrocarbon (alicyclic hydrocarbon).

Examples of the saturated aliphatic hydrocarbon include, but are not particularly limited to, a linear or branched saturated aliphatic hydrocarbon(alkane) having 1 to 22 carbon atoms, and specific examples thereof include, for example, methane, ethane, propane, butane, pentane, hexane, heptane, octane, nonane, decane, undecane, dodecane, tridecane, tetradecane, pentadecane, hexadecane, heptadecane, octadecane, nonadecane, icosane, heneicosane, and docosane.

Examples of the unsaturated aliphatic hydrocarbon include, but are not particularly limited to, a linear or branched unsaturated aliphatic hydrocarbon (an alkene or alkyne) that has a carbon-carbon double bond or carbon-carbon triple bond and 2 to 22 carbon atoms.

Examples of the aromatic ring-containing aliphatic hydrocarbon include, but are not particularly limited to, a saturated or unsaturated aliphatic hydrocarbon that contains an aromatic hydrocarbon. Preferable embodiments thereof include a saturated aliphatic hydrocarbon having 7 to 24 carbon atoms, where this number of carbon atoms includes the number of carbon atoms present in the aromatic hydrocarbon, and more preferred is a combination of an aromatic hydrocarbon having 6 to 14 carbon atoms and a saturated or unsaturated hydrocarbon having 1 to 18 carbon atoms. Specific examples thereof include, for example, methylbenzene (toluene), ethylbenzene, dimethylbenzene (xylene), propylbenzene, butylbenzene, pentylbenzene, hexylbenzene, heptylbenzene, octylbenzene, nonylbenzene, decylbenzene, undecylbenzene, dodecylbenzene, tridecylbenzene, tetradecylbenzene, pentadecylbenzene, hexadecylbenzene, heptadecylbenzene, octadecylbenzene, methylnaphthalene, ethylnaphthalene, and dimethylnaphthalene.

Examples of the aromatic hydrocarbon include, but are not particularly limited to, an aromatic hydrocarbon having 6 to 20 carbon atoms, and specific examples thereof include, for example, benzene, naphthalene, anthracene, naphthacene, and phenanthrene.

Examples of the aliphatic ring-containing aliphatic hydrocarbon include, but are not particularly limited to, a saturated or unsaturated aliphatic hydrocarbon that contains an aliphatic ring (cyclic hydrocarbon). Preferable embodiments thereof include a saturated aliphatic hydrocarbon having 7 to 24 carbon atoms, where this number of carbon atoms includes the number of carbon atoms present in the aliphatic ring, and more preferred is a combination of an aliphatic ring having 6 to 14 carbon atoms and a saturated or unsaturated hydrocarbon having 1 to 18 carbon atoms. Specific examples thereof include, for example, an aliphatic cyclic hydrocarbon as explained below to which methane, ethane, propane, butane, pentane, hexane, heptane, octane, nonane, decane, undecane, dodecane, tridecane, tetradecane, pentadecane, hexadecane, heptadecane, or octadecane is bonded. Examples of the aliphatic cyclic hydrocarbon include, but are not particularly limited to, a saturated or unsaturated aliphatic cyclic hydrocarbon having 3 to 14 carbon atoms, and specific examples thereof include, for example, cyclopropane, cyclobutane, cyclopentane, cyclohexane, cycloheptane, cyclooctane, cyclononane, cyclodecane, cycloundecane, cyclododecane, cyclotridecane, and cyclotetradecane

Examples of the sulfur-containing group include a group having a sulfur element and 0 to 18 carbon atoms, and specific examples thereof include, but are not particularly limited to, a thiophen-containing group, a thiazole-containing group, a thiol-containing group, a thioether-containing group, a thioalkoxy-containing group, a sulfo-containing group, a sulfide-containing group, a disulfide-containing group, thioester-containing group, a thioamide-containing group, a sulfonyl-containing group, a sulfo-containing group, a thiocarbonyl-containing group, a thiourea-containing group, a thiocarbamate-containing group, and a dithiocarbamate-containing group.

Examples of the oxygen-containing group include a group having an oxygen element and 0 to 18 carbon atoms, and specific examples thereof include, but are not particularly limited to, a hydroxy-containing group, an alkoxy-containing group, an acetyl-containing group, an aldehyde-containing group, a carboxy-containing group, an ether-containing group, a carbonyl-containing group, an ester-containing group, an amide-containing group, a nitro-containing group, a carbamate-containing group, a carbamoyl-containing group, a polyoxyethylene-containing group, and an oxo-containing group.

Examples of the nitrogen-containing group include a group having a nitrogen element and 0 to 18 carbon atoms, and specific examples thereof include, but are not particularly limited to, a cyano-containing group, a cyanato-containing group, an isocyanate-containing group, a nitro-containing group, a nitroalkyl-containing group, an amide-containing group, a urea-containing group, a urethane-containing group, an imide-containing group, a carbodiimide-containing group, an azo-containing group, a pyridine-containing group, an imidazole-containing group, a benzotriazole-containing group, an amino-containing group, a primary amino-containing group, a secondary amino-containing group, a tertiary amino-containing group, and an aminoalkyl-containing group.

Examples or preferred embodiments regarding the terms of hydrocarbon-containing group, sulfur-containing group, oxygen-containing group, and nitrogen-containing group-as used herein but excluding R¹ and R³ to R⁹-are interpreted based on the definitions above unless otherwise stated. When it is a monovalent group, the above definitions are applied as a group having one bond, for example, as a residue in which one hydrogen atom has been removed from a molecule. When it is a divalent group, the above definitions are applied as a group having two bonds, for example, as a residue in which two hydrogen atoms have been removed from a molecule.

It is preferred that each of R¹ and R³ to R⁵ be any one of a hydrogen atom, an oxygen-containing group, a hydrocarbon-containing group, and it is more preferred that it be any one of a hydrogen atom, a hydroxyl group, a hydrocarbon group, and a hydrocarbon-containing group having an oxygen-containing group, and it is even more preferred that it be any one of a hydrogen atom, a hydroxyl group, and a hydrocarbon group. It is preferred that the hydrocarbon group be a saturated aliphatic hydrocarbon group (alkyl group) having 1 to 8 carbon atoms, and it is more preferred that it be a saturated aliphatic hydrocarbon group having 1 to 4 carbon atoms, and it is even more preferred that it be a methyl group or a butyl group.

Preferable examples of the combinations of substituent groups for R¹ to R⁵ include the cases in which:
(A) R¹ is a hydroxyl group, R² is a hydrogen atom, R³ is a hydrogen atom, R⁴ is a hydrocarbon group, and R⁵ is a hydrogen atom;
(B) R¹ is a hydroxyl group, R² is a hydrogen atom, R³ is a hydrogen atom, R⁴ is a hydrogen atom, and R⁵ is a hydrogen atom;
(C) R¹ is a hydrogen atom, R² is a hydrogen atom, R³ is a hydrogen atom, R⁴ is a hydrocarbon group, and R⁵ is a hydroxyl group; and
(D) R¹ is a hydrogen atom, R² is a hydrogen atom, R³ is a hydrogen atom, R⁴ is a hydrocarbon-containing group having an oxygen-containing group, and R⁵ is a hydroxyl group.

It is preferred that each position of R⁶ to R⁹ that is not a monovalent sulfur-containing group having a sulfide bond be occupied by a hydrogen atom.

In the formula (1), at least one of R⁶ to R⁹ represents a monovalent sulfur-containing group that has a sulfide bond.

It is preferred that the monovalent sulfur-containing group that has a sulfide bond be positioned on either R⁷ or R⁸

It is preferred that the monovalent sulfur-containing group that has a sulfide bond be represented by a formula (2) defined as:
[Chem. 2]

-(R¹⁰)₁-S-(R¹¹-S)ₘ-R¹² (2)

In the formula (2), each of R¹⁰ and R¹¹ independently represents a divalent hydrocarbon-containing group, and R¹² represents a monovalent hydrocarbon-containing group. When m is two or more, the m R¹¹s may be different from or identical to one another.

In the formula (2), 1 represents an integer of 0 or 1, preferably of 0. m represents an integer of 0 to 3, preferably of 0 or 1, and more preferably of 0.

Examples of the divalent hydrocarbon-containing groups of R¹⁰ and R¹¹ include those as exemplified in the above.

It is preferred that each of R¹⁰ and R¹¹ be a hydrocarbon group, more preferably a saturated aliphatic hydrocarbon group having 1 to 22 carbon atoms.

Examples of the monovalent hydrocarbon-containing group of R¹² include those as exemplified in the above. Examples thereof also include a hydrocarbon-containing group having an oxygen-containing group to be explained below and a group represented by -A-S-R¹³.

In a preferable embodiment, R¹² is a hydrocarbon group. Preferable hydrocarbon groups include a saturated aliphatic hydrocarbon group, an aromatic ring-containing aliphatic hydrocarbon group, an aromatic hydrocarbon group, an aliphatic ring-containing aliphatic hydrocarbon group, and an aliphatic cyclic hydrocarbon group.

Examples of R¹² when it is a saturated aliphatic hydrocarbon group include, but are not particularly limited to, a group in which one hydrogen atom is removed from one of the saturated aliphatic hydrocarbons that are specifically exemplified in the above. It is preferred that the saturated aliphatic hydrocarbon group be a linear or branched group having 1 to 22 carbon atoms, more preferably a linear or branched group having 1 to 18 carbon atoms, even more preferably a linear or branched group having 1 to 12 carbon atoms, particularly preferably a linear group having 1 to 12 carbon atoms, and even more particularly preferably a linear group having 1 to 8 carbon atoms.

Examples of R¹² when it is an aromatic ring-containing aliphatic hydrocarbon group include, but are not particularly limited to, a group in which one hydrogen atom is removed from one of the aromatic ring-containing aliphatic hydrocarbons that are specifically exemplified in the above. The aromatic ring-containing saturated aliphatic hydrocarbon group is preferably a combination of an aromatic hydrocarbon having 6 to 14 carbon atoms and a saturated aliphatic hydrocarbon having 1 to 18 carbon atoms, more preferably a combination of a benzene ring and a saturated aliphatic hydrocarbon group having 1 to 8 carbon atoms, and even more preferably a combination of a benzene ring and a saturated aliphatic hydrocarbon group having 1 to 4 carbon atoms.

Examples of R¹² when it is an aromatic hydrocarbon group include, but are not particularly limited to, a group in which one hydrogen atom is removed from one of the aromatic hydrocarbons that are specifically exemplified in the above. Examples of the aromatic hydrocarbon group include aromatic hydrocarbons having 6 to 20 carbon atoms of which a phenyl group and a naphthyl group are preferred, and a phenyl group is more preferred.

Examples of R¹² when it is an aliphatic ring-containing aliphatic hydrocarbon group include, but are not particularly limited to, a group in which one hydrogen atom is removed from one of the aliphatic ring-containing aliphatic hydrocarbons that are specifically exemplified in the above. The aliphatic ring-containing aliphatic hydrocarbon group is preferably a combination of an aliphatic ring having 6 to 14 carbon atoms and a saturated or unsaturated hydrocarbon having 1 to 18 carbon atoms of which more preferred are a methylcyclohexyl group and an ethylcyclohexyl group.

Examples of R¹² when it is an aliphatic cyclic hydrocarbon include, but are not particularly limited to, a group in which one hydrogen atom is removed from one of the aliphatic cyclic hydrocarbons that are specifically exemplified in the above. Examples of the aliphatic cyclic hydrocarbon group include saturated or unsaturated alicyclic hydrocarbon groups having 3 to 12 carbon atoms of which preferred are a cyclopentyl group, a cyclohexyl group, a cyclohexenyl group, and a cyclooctyl group.

Another preferable embodiment is directed to the one in which R¹² is a hydrocarbon-containing group having an oxygen-containing group. Examples of the oxygen-containing group include the oxygen-containing groups as exemplified in the above. Of these, preferred are a hydroxy-containing group, an ether-containing group, a carbonyl-containing group, an ester-containing group, and an amide-containing group; more preferred are a hydroxy-containing group and an ester-containing group; and even more preferred is an ester-containing group. It is preferred that the oxygen-containing group be a group consisting of oxygen, carbon, and hydrogen atoms. The number of carbon atoms in the oxygen-containing group may be 3 to 36, preferably 3 to 24, and even more preferably 3 to 18.

Another preferable embodiment includes the one in which the oxygen-containing group is a reactive functional group. Examples of the reactive functional group include a polymerizable group that can serve as a raw material for resin and a functional group that can bond to an organic or inorganic material. When R¹² is a hydrocarbon-containing group having a reactive functional group, it can be bonded to, for example, an organic or inorganic material and serve as a raw material (monomer) for a resin member, thereby allowing the ultraviolet absorber to prevent bleeding out from the organic or inorganic material, and thus making it useful.

Examples of the reactive functional group include, but are not particularly limited to, a hydroxy-containing group, an ester-containing group, and a carboxy-containing group.

Examples of the ester-containing group include, but are not particularly limited to, a (meth)acryloyloxy group, a maleoyldioxy group, and a cinnamoyloxy group of which preferred is a (meth)acryloyloxy group.

Preferable examples of the hydrocarbon-containing group having a reactive functional group include those in which a reactive functional group is bonded to a hydrocarbon group.

Another preferable embodiment is directed to the one in which R¹² is a hydrocarbon-containing group that contains a nitrogen-containing group having an unsaturated bond or cyclic structure. Such nitrogen-containing group is preferable for absorbing ultraviolet rays.

Of these, more preferred is a hydrocarbon-containing group that contains a nitrogen-containing group having a cyclic structure, and preferable examples of the nitrogen-containing group having a cyclic structure include a pyridine-containing group, an imidazole-containing group, and a benzotriazole-containing group, of which more preferred is a benzotriazole-containing group.

It is preferred that the hydrocarbon-containing group that contains a benzotriazole-containing group be represented by -A-S-R¹³, wherein A is a divalent hydrocarbon-containing group, a divalent hydrocarbon-containing group having a sulfur-containing group, or a divalent hydrocarbon-containing group having an oxygen-containing group, and R¹³ is a benzotriazole-containing group be represented by a formula (3) as defined below.

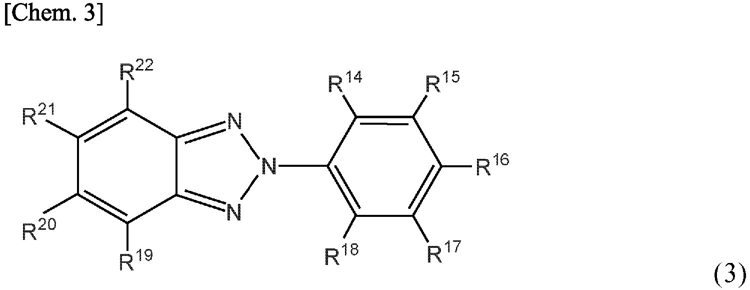

In the formula (3), each of R¹⁴ to R²² independently represents a hydrogen atom or a monovalent group selected from a hydrocarbon-containing group, a sulfur-containing group, a nitrogen-containing group, and an oxygen-containing group, and R¹⁵ represents a hydrogen atom. Any one of R¹⁴ and R¹⁶ to R²² is bonded to the sulfur atom in -A-S-R¹³,

Examples of the monovalent group selected from a hydrocarbon-containing group, a sulfur-containing group, a nitrogen-containing group, and an oxygen-containing group include those as exemplified above.

It is preferred that each position of R¹⁴ and R¹⁶ to R¹⁸ not bonded to the sulfur atom in -A-S-R¹³ be occupied by any one of a hydrogen atom, an oxygen-containing group, and a hydrocarbon-containing group of which preferred is any one of a hydrogen atom, a hydroxyl group, and a hydrocarbon group. The hydrocarbon group is preferably a saturated aliphatic hydrocarbon group (alkyl group) having 1 to 8 carbon atoms, more preferably a saturated aliphatic hydrocarbon group having 1 to 4 carbon atoms, and even more preferably a methyl group or a butyl group.

It is preferred that each position of R¹⁹ to R²² not bonded to the sulfur atom in -A-S-R¹³ be occupied by a hydrogen atom.

It is preferred that the position of R¹⁴ and R¹⁶ to R²² bonded to the sulfur atom in -AS-R¹³ be either R²⁰ or R²¹.

Examples of the divalent hydrocarbon-containing groups as A include those as exemplified in the above. Preferable hydrocarbon-containing groups include a saturated aliphatic hydrocarbon group, an aromatic ring-containing aliphatic hydrocarbon group, and an aromatic hydrocarbon group of which more preferred is a saturated aliphatic hydrocarbon group.

Examples of the divalent hydrocarbon-containing group having a sulfur-containing group as A include the above-exemplified hydrocarbon-containing groups having a sulfur-containing group. Such sulfur-containing group is preferably a thioether-containing group or a sulfide-containing group. Such hydrocarbon group is preferably a saturated aliphatic hydrocarbon group, an aromatic ring-containing aliphatic hydrocarbon group, or an aromatic hydrocarbon group.

Examples of the divalent hydrocarbon-containing group having an oxygen-containing group as A include the above-exemplified hydrocarbon-containing groups having an oxygen-containing group. Such oxygen-containing group is preferably a hydroxy-containing group, an ether-containing group, a carbonyl-containing group, an ester-containing group, or an amide-containing group.

Of the above-mentioned R¹²s, it is preferred in terms of achieving a light absorption peak having a steep slope to the long-wavelength side of the peak (the absolute value of the slope is large) that R¹² be a hydrocarbon-containing group having an oxygen-containing group, a saturated aliphatic hydrocarbon group, an aromatic ring-containing aliphatic hydrocarbon group, or an aromatic hydrocarbon group; more preferably a hydrocarbon-containing group having an oxygen-containing group, a saturated aliphatic hydrocarbon group, or an aromatic ring-containing aliphatic hydrocarbon group; and even more preferably a hydrocarbon-containing group having an oxygen-containing group or a saturated aliphatic hydrocarbon group.

### (Light absorption/transmittance effect)

The ultraviolet absorber according to the present invention has a structure represented by the formula (1) to thereby bring a technical advantage that it enhances the sharpness of the light absorption peak or transmittance curve (absolute value of the slope is large), and allows suppression of the transmission of wavelengths intended for absorption while efficiently transmitting wavelengths intended for transmission when these wavelengths intended for absorption and transmission are in close proximity. For example, a 0.075wt% toluene solution can achieve 1% transmittance at 400 nm and 90% transmittance at 415 nm, and adjustment of the additive amounts enables efficient absorption of light ranging from a long-wavelength ultraviolet light region (350 to 400 nm) to a short-wavelength visible light region (400 to 420 nm) while inhibiting the absorption of visible light (400 to 800 nm).

### (Characteristic of absorption peak)

The ultraviolet absorber according to the present invention exhibits a maximum light absorption wavelength of 350 to 390 nm in a chloroform solution having a predefined concentration and the absolute value of the slope is 0.060 or more, where the value represents the slope of a straight line connecting the maximum absorption wavelength to the point at which the absorption spectrum on the long-wavelength side reaches a light absorbance of 0.1. The above-mentioned predefined concentration is determined such that a compound having one benzotriazole frame structure is used as a solution of 100 µM while a compound having two benzotriazole frame structures is used as a solution of 50 µM.

It is preferred in terms of being capable of inhibiting light absorption in the visible light region that the maximum light absorption peak fall within the range of 350 to 370 nm, more preferably of 350 to 366 nm.

It is also preferred in terms of being capable of inhibiting light absorption in the visible light region that the absolute value of the slope be 0.065 or more, where the value represents the slope of a straight line connecting the maximum absorption wavelength to the point at which the absorption spectrum on the long-wavelength side reaches an absorbance of 0.1.

The ultraviolet absorber according to the present invention efficiently absorbs light in the 350 to 390 nm range and exhibits a large absolute value of the slope of the absorption peak on the long-wavelength side, which therefore inhibits the initial yellowing of thin members-such as resin films, resin sheets, glass films, and glass sheets-even when the absorber is added thereto at high concentrations, thereby capable of preserving their transparency.

### (Characteristic of transmittance curve)

The wavelength of the transmitted light in the light transmittance curve varies based on the measurement conditions (such as concentration and optical path length) in UV-Vis transmission spectroscopy, but it is preferred that the ultraviolet absorber according to the present invention demonstrates a maximum slope value of the light transmittance curve of 9.0 or greater in a toluene solution with a concentration ranging from 0.050wt% to 10wt%. The maximum slope value of the light transmittance curve can be determined as, for example, the largest differential value in transmittance (Δ%T/1 nm) across a wavelength interval of 1 nm. The larger the slope on the light transmittance curve is, the steeper the slope becomes on the long-wavelength side of the absorption peak (the larger the absolute value of the slope becomes), and adjusting the additive amount enables efficient absorption of light ranging from a long-wavelength ultraviolet light region (350 to 400 nm) to a short-wavelength visible light region (400 to 420 nm) while inhibiting the absorption of visible light (400 to 800 nm). Further, it enables the absorption of long-wavelength ultraviolet light while preventing the initial yellowing of a matrix by adjusting the additive amount when it is added into the matrix (organic or inorganic material).

### (Application of the ultraviolet absorber of the present invention)

The ultraviolet absorber of the present invention may be used for any purpose which is not particularly limited but it may be added to organic or inorganic materials for the purpose of, for example, inhibiting the transmission of light at specific wavelengths.

The ultraviolet absorber of the present invention has a high affinity, compatibility and reactivity to various organic or inorganic materials so that as a result of mixing, dissolving, dispersing, or reacting the ultraviolet absorber of the present invention, there can be obtained an uninform member; particularly, when employing a transparent member, there can be obtained a member superior in transparency (transparent member). Examples of such transparent member include, but are not particularly limited to, those made of resin (such as thermoplastic or thermosetting resin) or glass.

Examples of the organic material include, but are not particularly limited to, a resin, a material of plant/animal origin, and an organic compound such as a crude oil-derived material, while examples of such inorganic material include, but are not particularly limited to, a siliceous material made by a sol-gel method, glass, a glass (such as a silicon oxide, an alkali-free glass and a soda glass), a liquid glass (such as sodium silicate and potassium silicate), a low melting point glass, a quartz, a silicone resin, an alkoxysilane, a silane coupling agent, a metal, a metallic oxide, a mineral, and other such inorganic compounds. Of these, it may be suitably used to absorb or inhibit the transmission of light at 350 to 400 nm, and it is useful for, for example, improving light resistance, preventing resin deterioration, and inhibiting the initial yellowing of a transparent member made of, for example, glass or resin.

The ultraviolet absorber of the present invention is excellent in that it is less likely to evaporate or decompose under heat when added to a resin and used. The ultraviolet absorber of the present invention may be used alone, or two or more kinds thereof may be used in combination.

A conventional method may be applied to add the ultraviolet absorber to a resin. Applicable examples of such methods include, but are not particularly limited to, a method in which the ultraviolet absorber is mixed into a resin solution, a method in which a resin is melted and the ultraviolet absorber is mixed thereinto, and a method in which the ultraviolet absorber is mixed into a raw material monomer of the resin, followed by resinification. That is, the ultraviolet absorber according to the present invention may be added to a resin to form a resin composition.

As the resins to which the additive of the present invention is added, a conventionally known resin may be widely used, and examples thereof include, but are not particularly limited to, a thermoplastic resin and a thermosetting resin each including polymers having one kind of repeating unit as well as copolymers having multiple kinds of repeating unit.

Examples of the polymers of the thermoplastic resin include, but are not particularly limited to, a (meth)acryl-based resin (such as poly(meth)acrylic acid and methyl poly(meth)acrylate), an olefin-based resin (such as polyethylene and polypropylene), a styrene-based resin (such as polystyrene), an ester-based resin (such as polyethylene terephthalate resin), an ether-based resin (such as polyacetal), a vinyl chloride-based resin (such as polyvinyl chloride and polyvinylidene chloride), a fluorocarbon-based resin (such as polytetrafluoroethylene and polyvinyl fluoride), a vinyl-based resin (such as polyvinyl acetate and polyvinyl alcohol), a polycarbonate-based resin (such as polycarbonate), a polyamide-based resin (such as polyamide and nylon 6), a polyimide-based resin (such as such as polyimide), a polyamideimide-based resin (such as polyamideimide), a polymaleimide-based resin (such as polymaleimide), a polyvinylpyrrolidone-based resin (such as polyvinylpyrrolidone), a polyurethane-based resin (such as polyurethane), a polysulfone-based resin (such as polysulfone), a polyphenylene sulfide-based resin (such as polyphenylene sulfide), and a cycloolefin-based resin (such as a cycloolefin polymer).

Examples of the copolymers of the thermoplastic resin include, but are not particularly limited to, a butadiene-styrene-based copolymer (such as a butadiene-styrene copolymer), an acrylonitrile-styrene-based copolymer (such as an acrylonitrile-styrene copolymer), an acrylonitrile-butadiene-styrene-based copolymer (such as acrylonitrile-butadiene-styrene copolymer), a styrene-isoprene-based copolymer (such as a styrene-isoprene copolymer), a styrene-acrylic acid-based copolymer (such as a styrene-acrylic acid copolymer), and a vinyl chloride-vinylidene chloride-acrylonitrile-based copolymer (such as a vinyl chloride-vinylidene chloride-acrylonitrile copolymer).

Examples of the polymers of the thermosetting resin include, but are not particularly limited to, a phenol-based resin (such as a phenolic resin), a urea-based resin (such as a urea resin), a melamine-based resin (such as a melamine resin), an unsaturated polyester-based resin (such as an unsaturated polyester resin), an alkyd-based resin (such as an alkyd resin), an epoxy-based resin (such as an epoxy resin), and an episulfide-based resin (such as an episulfide resin).

Examples of the copolymers of the thermosetting resin include, but are not particularly limited to, an acrylic melamine-based resin (such as an acrylic melamine resin) and an acrylic urethane-based resin (such as an acrylic urethane resin).

When mixing and kneading, while performing heating, an ultraviolet absorber with an organic or inorganic substance such as a resin(s), or when processing or molding an ultraviolet absorber-containing resin member by heating, an additive having low heat resistance causes thermal decomposition or heat denaturation, fails to exhibit sufficient ultraviolet absorption efficiency, contaminates machinery, and impairs transparency of the resin when it is transparent. Additionally, thermal decomposition, heat denaturation, or similar processes reduce the transmittance in the visible light region and cause coloration. For this reason, desired is an ultraviolet absorber that inhibits coloration during heating, has a high decomposition onset temperature when it is heated, and exhibits excellent heat resistance.

The ultraviolet absorber according to the present invention exhibits excellent heat resistance (inhibits coloration during heating and has a high decomposition onset temperature when it is heated) and inhibits coloration under high temperature and functional deterioration of the additive, which thereby enabling the absorber to be used in a resin having a thermoforming or thermosetting temperature of 100°C or higher, particularly 200°C or higher, or preferably for the production or use at those temperatures. Accordingly, the ultraviolet absorber of the present invention may preferably be used in a polymer or copolymer of a thermoplastic or thermosetting resin, particularly in a polymer or copolymer of a thermoplastic or thermosetting resin having the above thermoforming temperature.

### Examples

Hereinbelow, the invention will be explained in more detail by means of working examples, but the present invention shall not be limited to these working examples.

### 1. Synthesis of ultraviolet absorber

Compounds 1 to 24 were synthesized by the methods as shown below.

### Intermediate 1

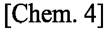

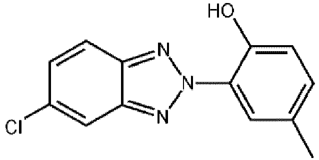

2-(2-hydroxy-3-tert-butyl-5-methylphenyl)-5-chlorobenzotriazole (59.2 g, 187 mmol) was dissolved in 500 mL of toluene at 80°C. Aluminum chloride anhydrous (50 g, 375 mmol) was slowly added thereinto. The mixture was kept at this temperature for 30 minutes to allow the reaction to proceed, and then cooled to room temperature. The reaction solution was added into 500 mL of cold water and the organic layer was washed three times. The organic layer was then condensed, and methanol was added to perform recrystallization, thereby obtaining an intermediate 1.

### Intermediate 2

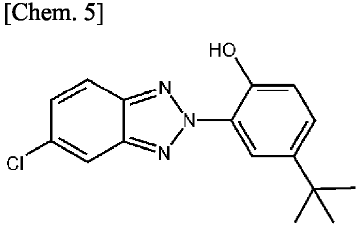

Into a mixture of 4-chloro-2-nitroaniline (17.2 g, 100 mmol), water (65 mL), and a 35% hydrochloric acid aqueous solution (43.34 g, 416 mmol) was delivered by drops a 36% sodium nitrite aqueous solution (69 g, 360 mmol) over 30 minutes under an ice-cooled condition, and the reaction was then allowed to proceed for one hour, resulting in an aqueous solution of diazonium salt. Next, the above-mentioned aqueous solution of diazonium salt was delivered under an ice-cooled condition by drops into a solution in which 4-tertbutylphenol (15.8 g,105 mmol), sodium hydroxide (7.68 g,192 mmol), and potassium carbonate (27.8 g, 201 mmol) were mixed with 80 g of water, and then the mixture was kept at this temperature for 1 hour to allow the reaction to proceed. After the reaction was over, hydrochloric acid was used to turn the reactant acidic, followed by collecting a red precipitate by filtration. The precipitate, a 25% NaOH aqueous solution (51 g), and a zinc powder (16.44 g, 251 mmol) were added in water (100m L) and they were reacted for 3 hours while being heated and refluxed. The mixture cooled to room temperature and then toluene and hydrochloric acid were added, followed by washing the organic layer three times, and then methanol was added, thereby obtaining an intermediate 2.

### Intermediate 3

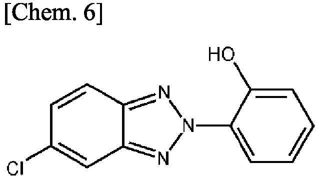

2-(3,5-di-tert-butyl-2-hydroxyphenyl)-5-chlorobenzotriazole (16.6 g, 46 mmol) was dissolved in 100 mL of toluene at 80°C. Aluminum chloride anhydrous (25 g, 187 mmol) was slowly added thereinto. The mixture was kept at this temperature for 3 hours to allow the reaction to proceed, and then cooled to room temperature. The reaction solution was added to 100 mL of cold water and the organic layer was washed three times. The organic layer was then condensed, and methanol was added to perform recrystallization, and then the resultant product was purified via column chromatography to thereby obtain an intermediate 3.

### Compound 1

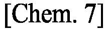

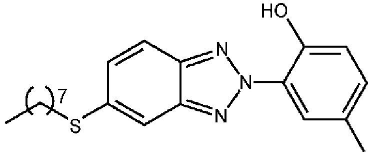

The intermediate 1 (5.0 g, 19 mmol), octanethiol (5.63 g, 38 mmol), potassium carbonate (5.85 g, 42 mmol), and potassium iodide (224 mg, 1.3 mmol) were reacted in 12.5 g of DMF at 135°C for 6 hours. After the reaction was completed, 12.5 g of water was added to precipitate a solid, which was then collected by filtration. The collected solid was dissolved in ethyl acetate followed by the addition of hydrochloric acid and water to adjust the pH of the water layer to 2 or less. The organic layer was washed with water three times and methanol was added to perform recrystallization, thereby obtaining a compound 1 of a white to light yellow solid.

FT-IR (ATR): 2917 cm⁻¹ : O-H stretching vibration, 1465, 1361 cm⁻¹ : triazole ring stretching vibration
¹HNMR (CDCl₃, 400 MHz): δ0.88(t, 3H, -CH₂-CH₃), 1.28-1.35(m, 8H, -CH₂-), 1.48(m, 2H, - CH₂-), 1.74(m, 2H, -CH₂-), 2.40(s, 3H, -CH₃), 3.04(t, 2H, -CH₂-S-), 7.09(d, 1H, CHₐᵣₒₘ), 7.14(d, 1H, CHₐᵣₒₘ), 7.37(d, 1H, CHₐᵣₒₘ), 7.70(s, 1H, CHₐᵣₒₘ), 7.81(d, 1H, CHₐᵣₒₘ), 8.16(s, 1H, CHₐᵣₒₘ), 11.00(s, 1H, -OH)
¹³C-NMR (CDCl₃:400 MHz): δ 14.1(-CH₂-CH₃), 20.5(-CH₃), 20.6, 22.6, 28.7, 29.0, 29.1, 31.8, 33.1(-CH₂-), 113.5, 117.6, 118.7, 121.0, 129.4, 131.1(CHₐᵣₒₘ), 124.8, 129.7, 138.3, 141.4, 143.6, 147.4 (Cₐᵣₒₘ)

### Compound 2

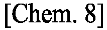

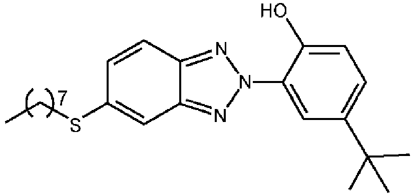

The intermediate 2 (2.0 g, 6.6 mmol), octanethiol (1.45 g, 10 mmol), potassium carbonate (2.0 g, 14 mmol), and potassium iodide (77 mg, 0.46 mmol) were reacted in 5 g of DMF at 135°C for 12 hours. After the reaction was completed, 5 g of water was added to precipitate a solid, which was then collected by filtration. The collected solid was dissolved in ethyl acetate followed by the addition of hydrochloric acid and water to adjust the pH of the water layer to 2 or less. The organic layer was washed with water three times and methanol was added to perform recrystallization, thereby obtaining a compound 2 of a white to light yellow solid.

FT-IR (ATR): 2979 cm⁻¹ : O-H stretching vibration, 1469, 1375 cm⁻¹ : triazole ring stretching vibration
¹HNMR (CDCl₃, 400 MHz): δ0.88(t, 3H, -CH₂-CH₃), 1.28-1.34(m, 8H, -CH₂-), 1.39(s, 9H, - C-(CH₃)₃), 1.48(m, 2H, -CH₂-), 1.74(m, 2H, -CH₂-), 3.04(t, 2H, -CH₂-S-), 7.13(d, 1H, CHₐᵣₒₘ), 7.35-7.39(m, 2H, CHₐᵣₒₘ), 7.71(s, 1H, CHₐᵣₒₘ), 7.81(d, 1H, CHₐᵣₒₘ), 8.36(d, 1H, CHₐᵣₒₘ), 11.06(s, 1H, -OH)
¹³C-NMR (CDCl₃:400 MHz): δ 14.1(-CH₂-CH₃), 22.7, 28.7, 29.0, 29.1, 31.4, 31.8, 33.1(-CH₂-), 31.5(-C-(CH₃)₃), 34.4(-C-(CH₃)₃), 113.5, 117.6, 118.5, 127.6, 129.3, 129.3(CHₐᵣₒₘ), 124.6, 138.2, 141.3, 143.3, 143.5, 147.3 (Cₐᵣₒₘ)

### Compound 3

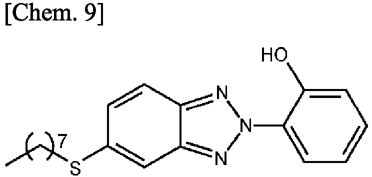

The intermediate 3 (1.40 g, 5.7 mmol), octanethiol (1.28 g, 8.7 mmol), potassium carbonate (1.73 g, 13 mmol), and potassium iodide (66 mg, 0.40 mmol) were reacted in 5 g of DMF at 135°C for 12 hours. After the reaction was completed, 5 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 3 of a light pink solid.

FT-IR (ATR): 2979 cm⁻¹ : O-H stretching vibration, 1464, 1375 cm⁻¹ : triazole ring stretching vibration
¹HNMR (CDCl₃, 400 MHz): δ0.88(t, 3H, -CH₂-CH₃), 1.28-1.35(m, 8H, -CH₂-), 1.48(m, 2H, - CH₂-), 1.74(m, 2H, -CH₂-), 3.04(t, 2H, -CH₂-S-), 7.05(t, 1H, CHₐᵣₒₘ), 7.20(d, 1H, CHₐᵣₒₘ), 7.32-7.39(m, 2H, CHₐᵣₒₘ), 7.70(s, 1H, CHₐᵣₒₘ), 7.81(d, 1H, CHₐᵣₒₘ), 8.35(d, 1H, CHₐᵣₒₘ), 11.23(s, 1H, -OH)
¹³C-NMR (CDCl₃:400 MHz): δ 14.1(-CH₂-CH₃), 22.6, 23.4, 28.7, 29.0, 29.1, 31.8, 33.1(-CH₂-), 113.5, 117.6, 119.0, 120.1, 121.0, 129.4, 130.3(CHₐᵣₒₘ), 125.4, 138.4, 141.4, 143.6, 149.6 (Cₐᵣₒₘ)

### Compound 4

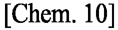

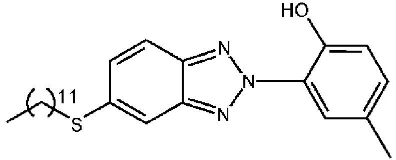

The intermediate 1 (5.0 g, 19 mmol), dodecanethiol (7.69 g, 38 mmol), potassium carbonate (5.85 g, 42 mmol), and potassium iodide (224 mg, 1.3 mmol) were reacted in 12.5 g of DMF at 135°C for 6 hours. After the reaction was completed, 12.5 g of water was added to precipitate a solid, which was then collected by filtration. The collected solid was dissolved in ethyl acetate followed by the addition of hydrochloric acid and water to adjust the pH of the water layer to 2 or less. The organic layer was washed with water three times and methanol was added to perform recrystallization, thereby obtaining a compound 4 of a white to light yellow solid.

FT-IR (ATR): 2917 cm⁻¹ : O-H stretching vibration, 1465, 1361 cm⁻¹ : triazole ring stretching vibration
¹HNMR (CDCl₃, 400 MHz): δ0.88(t, 3H, -CH₂-CH₃), 1.26-1.31(m, 16H, -CH₂-), 1.47(m, 2H, -CH₂-), 1.74(m, 2H, -CH₂-), 2.39(s, 3H, -CH₃), 3.03(t, 2H, -CH₂-S-), 7.08(d, 1H, CHₐᵣₒₘ), 7.14(d, 1H, CHₐᵣₒₘ), 7.36(d, 1H, CHₐᵣₒₘ), 7.69(s, 1H, CHₐᵣₒₘ), 7.80(d, 1H, CHₐᵣₒₘ), 8.15(s, 1H, CHₐᵣₒₘ), 11.00(s, 1H, -OH)
¹³C-NMR (CDCl₃:400 MHz): δ 14.2(-CH₂-CH₃), 20.5(-CH₃), 20.6, 22.7, 28.7, 29.0, 29.2, 29.4, 29.5, 29.6, 29.6, 31.9, 33.1(-CH₂-), 113.5, 117.6, 118.7, 120.9, 129.4, 131.1(CHₐᵣₒₘ), 124.8, 129.7, 138.3, 141.4, 143.6, 147.4 (Cₐᵣₒₘ)

### Compound 5

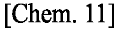

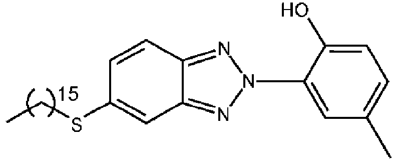

The intermediate 1 (5.0 g, 19 mmol), hexadecanethiol (9.82 g, 38 mmol), potassium carbonate (5.85 g, 42 mmol), and potassium iodide (224 mg, 1.3 mmol) were reacted in 12.5 g of DMF at 135°C for 6 hours. After the reaction was completed, 12.5 g of water was added to precipitate a solid, which was then collected by filtration. The collected solid was dissolved in ethyl acetate followed by the addition of hydrochloric acid and water to adjust the pH of the water layer to 2 or less. The organic layer was washed with water three times and methanol was added to perform recrystallization, thereby obtaining a compound 5 of a white to light yellow solid.

FT-IR (ATR): 2977 cm⁻¹ : O-H stretching vibration, 1443, 1374 cm⁻¹ : triazole ring stretching vibration
¹HNMR (CDCl₃, 400 MHz): δ0.88(t, 3H, -CH₂-CH₃), 1.25-1.30(m, 24H, -CH₂-), 1.48(m, 2H, -CH₂-), 1.74(m, 2H, -CH₂-), 2.39(s, 3H, -CH₃), 3.03(t, 2H, -CH₂-S-), 7.08(d, 1H, CHₐᵣₒₘ), 7.14(d, 1H, CHₐᵣₒₘ), 7.37(d, 1H, CHₐᵣₒₘ), 7.70(s, 1H, CHₐᵣₒₘ), 7.80(d, 1H, CHₐᵣₒₘ), 8.15(s, 1H, CHₐᵣₒₘ), 11.00(s, 1H, -OH)
¹³C-NMR (CDCl₃:400 MHz): δ 14.2(-CH₂-CH₃), 20.5(-CH₃), 20.6, 22.7, 28.7, 29.0, 29.2, 29.4, 29.5, 29.6, 29.7, 31.9, 33.1(-CH₂-), 113.5, 117.6, 118.7, 120.9, 129.4, 131.1(CHₐᵣₒₘ), 124.8, 129.7, 138.3, 141.4, 143.6, 147.4 (Cₐᵣₒₘ)

### Compound 6

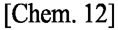

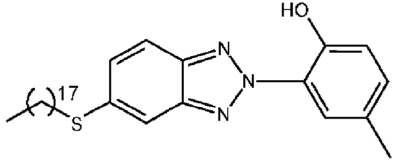

The intermediate 1 (5.0 g, 19 mmol), octadecanethiol (10.9 g, 38 mmol), potassium carbonate (5.85 g, 42 mmol), and potassium iodide (224 mg, 1.3 mmol) were reacted in 12.5 g of DMF at 135°C for 6 hours. After the reaction was completed, 12.5 g of water was added to precipitate a solid, which was then collected by filtration. The collected solid was dissolved in ethyl acetate followed by the addition of hydrochloric acid and water to adjust the pH of the water layer to 2 or less. The organic layer was washed with water three times and methanol was added to perform recrystallization, and then the resultant product was purified via column chromatography to thereby obtain a compound 6 of a white to light yellow solid.

### Compound 7

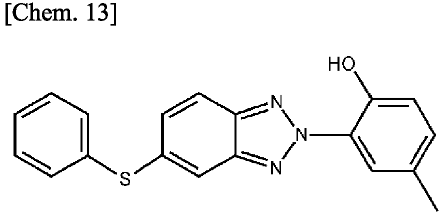

The intermediate 1 (5.0 g, 19 mmol), benzenethiol (2.76 g, 25 mmol), potassium carbonate (5.85 g, 42 mmol), and potassium iodide (224 mg, 1.3 mmol) were reacted in 12.5 g of DMF at 135°C for 6 hours. After the reaction was completed, 12.5 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 7 of a white to light yellow solid.

FT-IR (ATR): 2978 cm⁻¹ : O-H stretching vibration, 1440, 1373 cm⁻¹ : triazole ring stretching vibration
¹HNMR (CDCl₃, 400 MHz): δ2.39(s, 3H, -CH₃), 7.08(d, 1H, CHₐᵣₒₘ), 7.15(d, 1H, CHₐᵣₒₘ), 7.36-7.42(m, 4H, CHₐᵣₒₘ), 7.48-7.50(m, 2H, CHₐᵣₒₘ), 7.70(s, 1H, CHₐᵣₒₘ), 7.83(d, 1H, CHₐᵣₒₘ), 8.14(s, 1H, CHₐᵣₒₘ), 10.93(s, 1H, -OH)
¹³C-NMR (CDCl₃:400 MHz): δ 20.6(-CH₃), 116.8, 118.1, 118.8, 121.0, 128.3, 129.7, 129.9, 130.0, 132.8(CHₐᵣₒₘ), 124.8, 129.7, 133.6, 137.6, 141.8, 143.4, 147.4 (Cₐᵣₒₘ)

### Compound 8

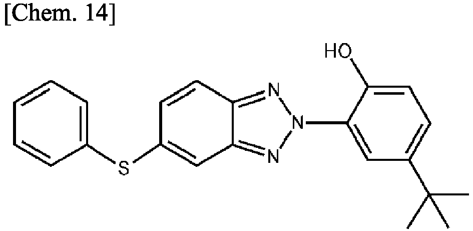

The intermediate 2 (2.0 g, 6.6 mmol), benzenethiol (876 mg, 7.9 mmol), potassium carbonate (2.0 g, 14 mmol), and potassium iodide (77 mg, 0.46 mmol) were reacted in 5 g of DMF at 135°C for 12 hours. After the reaction was completed, 5 g of water was added to precipitate a solid, which was then collected by filtration. The collected solid was dissolved in ethyl acetate followed by the addition of hydrochloric acid and water to adjust the pH of the water layer to 2 or less. The organic layer was washed with water three times and methanol was added to perform recrystallization, thereby obtaining a compound 8 of a white to light yellow solid.

FT-IR (ATR): 2979 cm⁻¹ : O-H stretching vibration, 1464, 1375 cm⁻¹ : triazole ring stretching vibration
¹HNMR (CDCl₃, 400 MHz): δ1.38(s, 9H, -C-(CH₃)₃), 7.12(d, 1H, CHₐᵣₒₘ), 7.37-7.42(m, 5H, CHₐᵣₒₘ), 7.48-7.51(m, 2H, CHₐᵣₒₘ), 7.71(s, 1H, CHₐᵣₒₘ), 7.84(d, 1H, CHₐᵣₒₘ), 8.35(s, 1H, CHₐᵣₒₘ), 10.99(s, 1H, -OH)
¹³C-NMR (CDCl₃:400 MHz): δ31.5(-CH₃), 34.4(-C-(CH₃)₃), 116.8, 117.7, 118.1, 118.6, 127.9, 128.3, 129.6, 129.8, 132.8(CHₐᵣₒₘ), 122.1, 124.6, 133.6, 137.5, 141.8, 143.4, 147.4 (Cₐᵣₒₘ)

### Compound 9

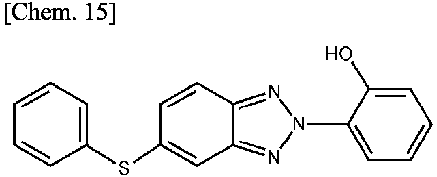

The intermediate 3 (1.40 g, 5.7 mmol), benzenethiol (754 mg, 6.8 mmol), potassium carbonate (1.73 g, 13 mmol), and potassium iodide (66 mg, 0.40 mmol) were reacted in 5 g of DMF at 135°C for 12 hours. After the reaction was completed, 5 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 9 of a light pink solid.

FT-IR (ATR):
¹HNMR (CDCl₃, 400 MHz): δ7.04(t, 1H, CHₐᵣₒₘ), 7.19(d, 1H, CHₐᵣₒₘ), 7.32-7.43(m, 5H, CHₐᵣₒₘ), 7.48-7.51(m, 2H, CHₐᵣₒₘ), 7.71(s, 1H, CHₐᵣₒₘ), 7.84(d, 1H, CHₐᵣₒₘ), 8.34(d, 1H, CHₐᵣₒₘ), 11.16(s, 1H, -OH)
¹³C-NMR (CDCl₃:400 MHz): δ 20.6(-CH₃), 116.8, 116.8, 118.1, 118.8, 118.8, 121.0, 128.3, 129.7, 129.9, 130.0, 132.8(CHₐᵣₒₘ), 124.8, 129.7, 133.6, 137.6, 141.8, 143.4, 147.4 (Cₐᵣₒₘ)

### Compound 10

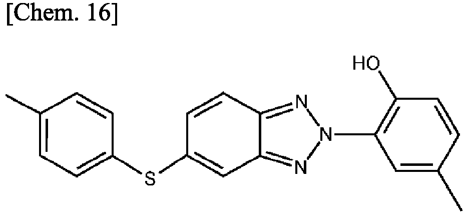

The intermediate 1 (5.0 g, 19 mmol), 4-toluenethiol (3.11 g, 25 mmol), potassium carbonate (5.85 g, 42 mmol), and potassium iodide (224 mg, 1.3 mmol) were reacted in 12.5 g of DMF at 135°C for 6 hours. After the reaction was completed, 12.5 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 10 of a white to light yellow solid.

FT-IR (ATR): 2978 cm⁻¹ : O-H stretching vibration, 1447, 1374 cm⁻¹ : triazole ring stretching vibration
¹HNMR (CDCl₃, 400 MHz): δ2.38(s, 3H, -CH₃), 2.40(s, 3H, -CH₃), 7.07(d, 1H, CHₐᵣₒₘ), 7.14(d, 1H, CHₐᵣₒₘ), 7.23(d, 2H, CHₐᵣₒₘ), 7.36(dd, 1H, CHₐᵣₒₘ), 7.43(d, 2H, CHₐᵣₒₘ), 7.57(s, 1H, CHₐᵣₒₘ), 7.80(d, 2H, CHₐᵣₒₘ), 8.13(s, 1H, CHₐᵣₒₘ), 10.95(s, 1H, -OH)
¹³C-NMR (CDCl₃:400 MHz): δ 20.5, 21.3(-CH₃), 115.3, 117.9, 118.8, 121.0, 129.2, 130.6, 131.2, 133.8(CHₐᵣₒₘ), 115.3, 124.8, 129.7, 138.9, 139.0, 141.6, 143.4, 147.4 (Cₐᵣₒₘ)

### Compound 11

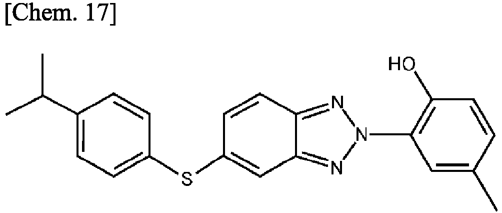

The intermediate 1 (5.0 g, 19 mmol), 4-isopropylbenzenethiol (3.81 g, 25 mmol), potassium carbonate (5.85 g, 42 mmol), and potassium iodide (224 mg, 1.3 mmol) were reacted in 12.5 g of DMF at 135°C for 6 hours. After the reaction was completed, 12.5 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 11 of a white to light yellow solid.

FT-IR (ATR): 2978 cm⁻¹ : O-H stretching vibration, 1447, 1374 cm⁻¹ : triazole ring stretching vibration
¹HNMR (CDCl₃, 400 MHz): δ2.38(s, 3H, -CH₃), 2.40(s, 3H, -CH₃), 7.07(d, 1H, CHₐᵣₒₘ), 7.14(d, 1H, CHₐᵣₒₘ), 7.23(d, 2H, CHₐᵣₒₘ), 7.36(dd, 1H, CHₐᵣₒₘ), 7.43(d, 2H, CHₐᵣₒₘ), 7.57(s, 1H, CHₐᵣₒₘ), 7.80(d, 2H, CHₐᵣₒₘ), 8.13(s, 1H, CHₐᵣₒₘ), 10.95(s, 1H, -OH)
¹³C-NMR (CDCl₃:400 MHz): δ 20.5, 23.9(-CH₃), 33.9(-CH-), 115.4, 117.9, 118.8, 121.0, 127.9, 129.4, 131.2, 133.6(CHₐᵣₒₘ), 124.8, 129.5, 129.7, 138.8, 141.6, 143.5, 147.4, 149.8(Cₐᵣₒₘ)

### Compound 12

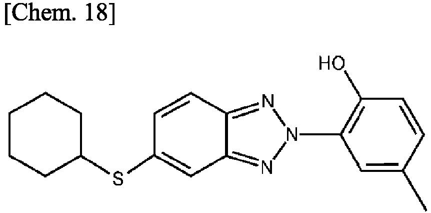

The intermediate 1 (20.0 g, 77 mmol), cyclohexanethiol (11.64 g, 100 mmol), potassium carbonate (23.4 g, 169 mmol), and potassium iodide (896 mg, 5.4 mmol) were reacted in 50 g of DMF at 135°C for 6 hours. After the reaction was completed, 50 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 12 of a white to light yellow solid.

### Compound 13

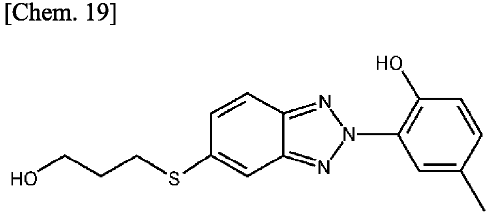

The intermediate 1 (18.2 g, 70 mmol), 3-mercapto-1-propanol (11.6 g, 126 mmol), potassium carbonate (21.3 g, 154 mmol), and potassium iodide (812 mg, 4.9 mmol) were reacted in 45 g of DMF at 135°C for 12 hours. After the reaction was completed, 45 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 13 of a white to light yellow solid.

FT-IR (ATR): 3735, 2978 cm⁻¹ : O-H stretching vibration, 1453, 1375 cm⁻¹ : triazole ring stretching vibration
¹HNMR (CDCl₃, 400 MHz): õ1.48(t, 1H, -OH), 2.00(m, 2H, -CH₂-CH₂-CH₂-), 3.18(t, 2H, - CH₂-S-), 3.84(q, 2H, HO-CH₂-), 7.08(d, 1H, CHₐᵣₒₘ), 7.16(d, 1H, CHₐᵣₒₘ), 7.39(d, 1H, CHₐᵣₒₘ), 7.76(s, 1H, CHₐᵣₒₘ), 7.82(d, 1H, CHₐᵣₒₘ), 8.16(s, 1H, CHₐᵣₒₘ), 10.99(s, 1H, -OH)
¹³C-NMR (CDCl₃:400 MHz): δ 20.6(-CH₃), 29.7, 31.3, 61.3(-CH₂-), 114.1, 117.8, 118.8, 121.0, 129.4, 131.2(CHₐᵣₒₘ), 124.8, 129.7, 137.5, 141.4, 143.5, 147.4 (Cₐᵣₒₘ)

### Compound 14

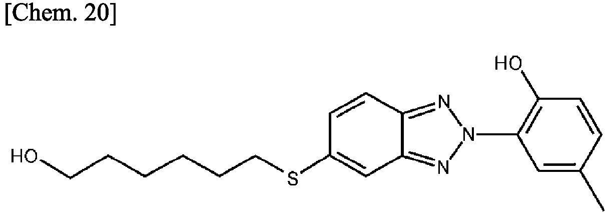

The intermediate 1 (51.9 g, 200 mmol), 6-mercapto-1-hexanol (34.9 g, 260 mmol), potassium carbonate (60.8 g, 440 mmol), and potassium iodide (2.32 g, 14 mmol) were reacted in 130 g of DMF at 135°C for 12 hours. After the reaction was completed, 130 g of water was added to precipitate a solid, which was then collected by filtration. The collected solid was dissolved in toluene followed by the addition of hydrochloric acid and water to adjust the pH of the water layer to 2 or less. The organic layer was washed with water three times and methanol was added to perform recrystallization, thereby obtaining a compound 14 of a white to light yellow solid.

### Compound 15

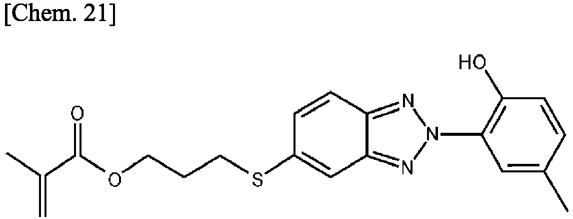

The compound 13 (20 g, 63 mmol), methacrylic acid (6.55 g, 76 mmol), and p-toluenesulfonic acid monohydrate (1.20 g, 6.3 mmol) were stirred under reflux for 5 hours in 50 g of toluene while removing water produced therein. After the reaction was completed, the organic layer was washed with water three times and methanol was added to perform purification, thereby obtaining a compound 15 of a white to light yellow solid.

FT-IR (ATR): 2978 cm⁻¹ : O-H stretching vibration, 1713 cm⁻¹ : C=O stretching vibration, 1452, 1375 cm⁻¹ : triazole ring stretching vibration, 1197 cm⁻¹ : C-O stretching vibration
¹HNMR (CDCl₃, 400 MHz): δ1.97(t, 3H, -C(=CH₂)-CH₃), 2.12(m, 2H, -CH₂-), 2.40(s, 3H, - CH₃), 3.14(t, 2H, -CH₂-), 4.31(t, 2H, -CH₂-), 5.60(t, 1H, =CH₂), 6.14(s, 1H, =CH₂), 7.09(d, 1H, CHₐᵣₒₘ), 7.16(d, 1H, CHₐᵣₒₘ), 7.39(d, 1H, CHₐᵣₒₘ), 7.76(s, 1H, CHₐᵣₒₘ), 7.82(d, 1H, CHₐᵣₒₘ), 8.16(s, 1H, CHₐᵣₒₘ), 10.98(s, 1H, -OH)
¹³C-NMR (CDCl₃:400 MHz): δ 18.4(-C(=CH₂)-CH₃), 20.6(-CH₃), 28.0, 29.8, 63.0(-CH₂-), 114.4, 117.9, 118.8, 121.0, 129.5, 131.3(CHₐᵣₒₘ), 124.8, 129.7, 137.1, 141.5, 143.5, 147.4 (Cₐᵣₒₘ), 125.7(=CH₂), 136.2(-C=CH₂), 167.3(C=O)

### Compound 16

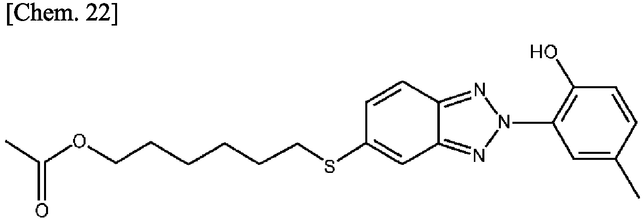

The compound 14 (11.6 g, 32 mmol), acetic acid (2.34 g, 39 mmol), and p-toluenesulfonic acid monohydrate (617 mg, 3.2 mmol) were stirred under reflux for 12 hours in 25 g of toluene while removing water produced therein. After the reaction was completed, the organic layer was washed with water three times and methanol was added to perform recrystallization, thereby obtaining a compound 16 of a white to light yellow solid.

### Compound 17

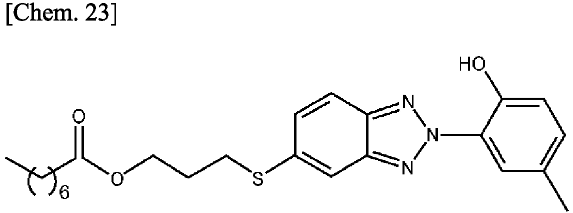

The compound 13 (18.2 g, 70 mmol), octane acid (11.6 g, 126 mmol), and p-toluenesulfonic acid monohydrate (812 mg, 4.9 mmol) were stirred under reflux for 3 hours in 40 g of toluene while removing water produced therein. After the reaction was completed, the organic layer was washed with water three times and then the resultant product was purified via column chromatography to thereby obtain a compound 17 of a white solid.

FT-IR (ATR): 2978 cm⁻¹ : O-H stretching vibration, 1739 cm⁻¹ : C=O stretching vibration, 1452, 1375 cm⁻¹ : triazole ring stretching vibration, 1207 cm⁻¹ : C-O stretching vibration ¹HNMR (CDCl₃, 400 MHz): δ0.86(t, 3H, -CH₂-CH₃), 1.26-1.30(m, 8H, -CH₂-), 1.63(m, 2H, - CH₂-), 2.06 (m, 2H, -CH₂-), 2.33(t, 2H, -CH₂-C(=O)-O-), 2.40(s, 3H, -CH₃), 3.11(t, 2H, -CH₂-), 3.11(t, 2H, -CH₂-S-), 4.23(t, 2H, -O-CH₂-), 7.09(d, 1H, CHₐᵣₒₘ), 7.15(d, 1H, CHₐᵣₒₘ), 7.39(d, 1H, CHₐᵣₒₘ), 7.76(s, 1H, CHₐᵣₒₘ), 7.83(d, 1H, CHₐᵣₒₘ), 8.16(s, 1H, CHₐᵣₒₘ), 10.97(s, 1H, -OH) ¹³C-NMR (CDCl₃:400 MHz): δ 14.1(-CH₂-CH₃), 20.5(-CH₃), 22.6, 25.0, 28.0, 28.9, 29.1, 29.8, 31.7, 34.3, 62.5(-CH₂-), 114.5, 117.9, 118.8, 121.0, 129.5, 131.2(CHₐᵣₒₘ), 124.8, 129.7, 137.1, 141.5, 143.5, 147.4 (Cₐᵣₒₘ), 173.8(-C(=O)-)

### Compound 18

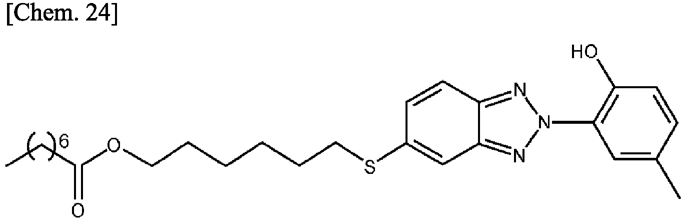

The compound 14 (21.7 g, 61 mmol), octane acid (13.2 g, 92 mmol), and p-toluenesulfonic acid monohydrate (1.16 g, 6.1 mmol) were stirred under reflux for 12 hours in 50 g of toluene while removing water produced therein. After the reaction was completed, the organic layer was washed with water three times and methanol was added to perform recrystallization, thereby obtaining a compound 18 of a white to light yellow solid.

### Compound 19

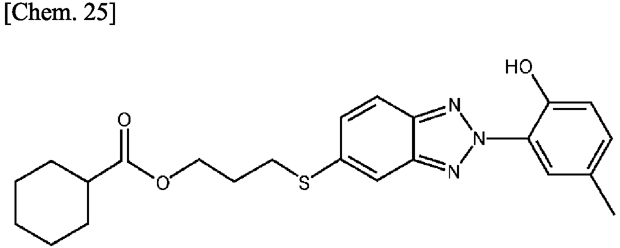

The compound 13 (19.2 g, 61 mmol), cyclohexanecarboxylic acid (11.8 g, 92 mmol), and p-toluenesulfonic acid monohydrate (1.16 g, 6.1 mmol) were stirred under reflux for 12 hours in 50 g of toluene while removing water produced therein. After the reaction was completed, the organic layer was washed with water three times and methanol was added to perform recrystallization, thereby obtaining a compound 19 of a white to light yellow solid.

### Compound 20

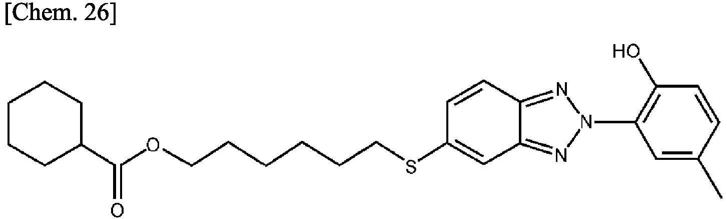

The compound 14 (21.7 g, 61 mmol), cyclohexanecarboxylic acid (11.8 g, 92 mmol), and p-toluenesulfonic acid monohydrate (1.16 g, 6.1 mmol) were stirred under reflux for 12 hours in 50 g of toluene while removing water produced therein. After the reaction was completed, the organic layer was washed with water three times and methanol was added to perform recrystallization, thereby obtaining a compound 20 of a white to light yellow solid.

### Compound 21

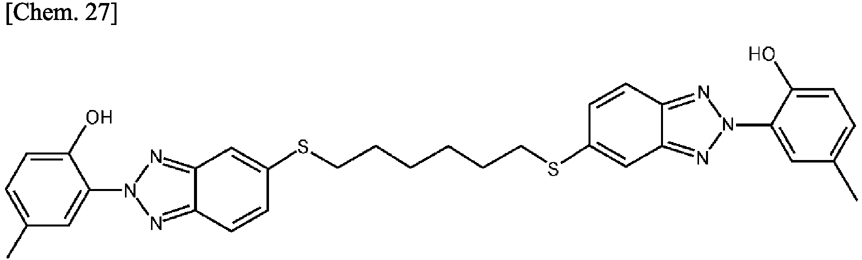

The intermediate 1 (13.0 g, 50 mmol), 1,6-hexanedithiol (3.43 g, 23 mmol), potassium carbonate (13.8 g, 100 mmol), and potassium iodide (581 mg, 3.5 mmol) were reacted in 23 g of DMF at 135°C for 12 hours. After the reaction was completed, 45 g of water was added to precipitate a solid, which was then collected by filtration. The collected solid was dissolved in toluene followed by the addition of hydrochloric acid and water to adjust the pH of the water layer to 2 or less. The organic layer was washed with water three times and it underwent recrystallization, thereby obtaining a compound 21 of a white to light yellow solid.

FT-IR (ATR): 2979 cm⁻¹ : O-H stretching vibration, 1461, 1387 cm⁻¹ : triazole ring stretching vibration
¹HNMR (CDCl₃, 400 MHz): δ1.55(m, 4H, -CH₂-CH₂-CH₂-S-), 1.77(m, 4H, -CH₂-CH₂-S-), 2.39(s, 6H, -CH₃), 3.04(t, 4H, -CH₂-S-), 7.08(d, 2H, CHₐᵣₒₘ), 7.14(d, 2H, CHₐᵣₒₘ), 7.36(d, 2H, CHₐᵣₒₘ), 7.70(s, 2H, CHₐᵣₒₘ), 7.08(d, 2H, CHₐᵣₒₘ), 8.15(s, 2H, CHₐᵣₒₘ), 10.99(s, 2H, -OH) ¹³C-NMR (CDCl₃:400 MHz): δ 20.5(-CH₃), 28.4, 28.5, 33.0(-CH₂-), 113.7, 117.7, 118.8, 120.9, 129.4, 131.2(CHₐᵣₒₘ), 124.8, 129.7, 138.0, 141.4, 143.5, 147.4 (Cₐᵣₒₘ)

### Compounds 22 and 23

The compounds 22 and 23 were synthesized in accordance with the synthesis method as set forth in WO-A-2016/021664.

### Compound 24

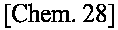

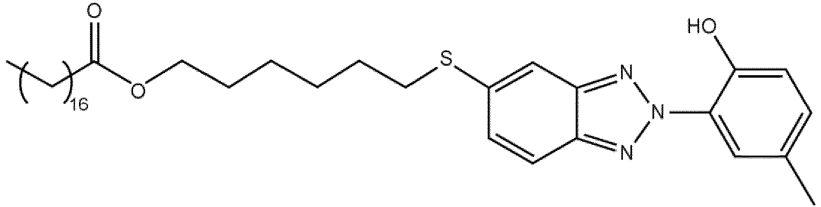

The compound 14 (3.6 g, 10 mmol), stearic acid (2.8 g, 10 mmol), and p-toluenesulfonic acid monohydrate (0.01 g, 0.1 mmol) were stirred under reflux for 4 hours in 50 g of toluene while removing water produced therein. After the reaction was completed, the organic layer was washed with water three times and methanol was added to perform recrystallization, thereby obtaining a compound 24 of a white to light yellow solid.
FT-IR (ATR): 2918 cm-1 : O-H stretching vibration, 1513, 1472 cm-1: triazole ring stretching vibration
1HNMR (CDCl3, 400 MHz): δ0.88-0.92(t, 3H,-CH3), 1.27(m, 30H, -CH2-),1.39-1.47 (m, 2H, -CH2-), 1.51-1.58 (m, 2H, -CH2-), 1.61-1.71 (m, 2H, -CH2-), 1.75-1.82 (m, 2H, -CH2-), 2.29-2.33 (t, 2H, -CH2S-), 2.42 (s, 3H, PhCH3), 3.05-3.09 (t, 2H, -CH2S-), 4.07-4.11 (t, 2H, -CH2S-) 7.10-7.12 (d, 2H, Carom), 7.16-7.19 (d, 1H, Carom), 7.38-7.41 (d, 1H, Carom), 7.73 (s, 1H, Carom), 7.83-7.85 (d, 1H, Carom), 8.19 (s, 1H, Carom), 11.02 (s, 1H, OH)
13C-NMR (CDCl3:400 MHz): δ 22.7, 25.0, 18.5, 29.3, 29.7, 33.0, 34.4, 64.1 (113.7, 117.7, 120.9, 121.0, 124.8, 129.3, 129.7, 131.2, 138.1, 141.4, 143.6, 147.4 (Carom), 174.0 (C=O)

### 2. UV-Vis absorption spectrum

UV-Vis absorption spectra of the compounds 1 to 11, 13, 15, 17, 18, and 21 to 24 were measured in a manner as shown below.

As to the compounds 1 to 11, 13, 15, 17, 18, and 22 to 24, 100 µM chloroform solutions of the respective compounds were prepared, while, as to the compound 21, 50 µM chloroform solution thereof was prepared, and an ultraviolet-visual-infrared spectrophotometer (UH41 50V by Hitachi High-Tech Science Corporation) was used to measure the absorption spectra thereof from which a wavelength at the maximum absorption peak was then read.

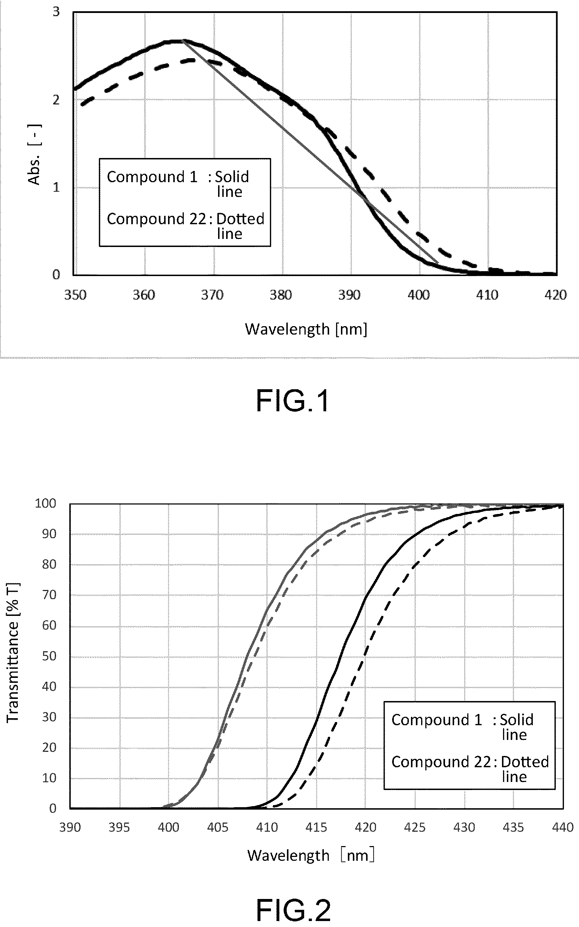

Measurement results are as shown in Table 1. FIG. 1 illustrates UV-Vis light absorption spectra of those of Working Example 1 (Solid line) and Comparative Example 1 (Dotted line).

The results for the compounds 1 to 24 indicate that a structure containing 2-phenylbenzotriazole with a monovalent sulfur-containing group having a sulfide bond resulted in a maximum absorption wavelength within the range of 350 to 370 nm. Each of the compounds 1 to 24 according to the present invention, where R² in the general formula (1) is a hydrogen atom, exhibited a maximum absorption wavelength within the range of 350 to 366 nm, and this shorter wavelength of the maximum absorption wavelength, compared to the compounds 22 and 23 (Comparative Examples 1 and 2), indicates their superior ability to reduce light absorption in the visible light spectrum.

**[Table 1A]**

| | | Structural formula | R¹ | R² | R³ | R⁴ | R⁵ | R⁶ | R⁷ | R⁸ | R⁹ | Maximum absorption wavelength (nm) | Molar extinction coefficient | Temperature at 5% weight loss (°C) |
|---|---|---|---|---|---|---|---|---|---|---|---|---|---|---|
| Working example 1 | Compound 1 | 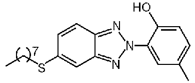 | OH | H | H | Me | H | H | C8H17S | H | H | 366 | 26670 | 277 |
| Working example 2 | Compound 2 | 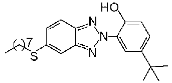 | OH | H | H | tBu | H | H | C8H17S | H | H | 365 | 26020 | 270 |
| Working example 3 | Compound 3 | 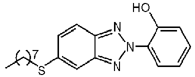 | OH | H | H | H | H | H | C8H17S | H | H | 361 | 26160 | 263 |
| Working example 4 | Compound 4 | 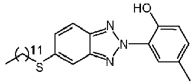 | OH | H | H | Me | H | H | C12H23S | H | H | 365 | 26950 | 288 |
| Working example 5 | Compound 5 | 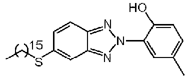 | OH | H | H | Me | H | H | C16H31S | H | H | 365 | 26270 | 327 |
| Working example 6 | Compound 6 | 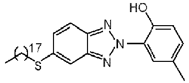 | OH | H | H | Me | H | H | C18H35S | H | H | 365 | 25740 | 338 |
| Working example 7 | Compound 7 | 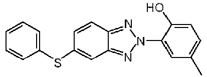 | OH | H | H | Me | H | H | PheS | H | H | 363 | 25520 | 271 |
| Working example 8 | Compound 8 | 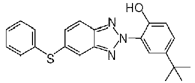 | OH | H | H | tBu | H | H | PheS | H | H | 363 | 25490 | 290 |
| Working example 9 | Compound 9 | 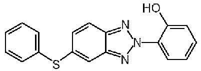 | OH | H | H | H | H | H | PheS | H | H | 360 | 26130 | 265 |
| Working example 10 | Compound 10 | 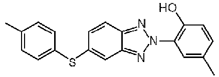 | OH | H | H | Me | H | H | 4-MePlieS | H | H | 366 | 27910 | 283 |
| Working example 11 | Compound 11 | 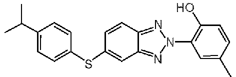 | OH | H | H | Me | H | H | 4-iPrPheS | H | H | 365 | 26950 | 296 |

**[Table 1B]**

| | | Structural formula | R¹ | R² | R³ | R⁴ | R⁵ | R⁶ | R⁷ | R⁸ | R⁹ | Maximum absorption wavelength (nm) | Molar Absorption Coefficient | 5% weight reduction temperature (°C) |
|---|---|---|---|---|---|---|---|---|---|---|---|---|---|---|
| Working example 12 | Compound 13 | 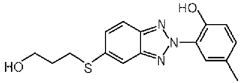 | OH | H | H | Me | H | H | HOC3H6S | H | H | 364 | 25480 | 288 |
| Working example 13 | Compound 15 | 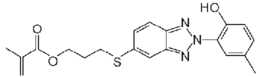 | OH | H | H | Me | H | H | Methacrylate -C3H6S | H | H | 365 | 26200 | 287 |
| Working example 14 | Compound 17 | 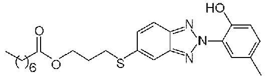 | OH | H | H | Me | H | H | C7H15COOC3H6S | H | H | 363 | 28360 | 308 |
| Working example 15 | Compound 18 | 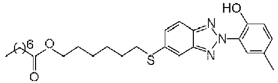 | OH | H | H | Me | H | H | C7H15COOC6H12S | H | H | 365 | 25030 | 326 |
| Working example 16 | Compound 21 | 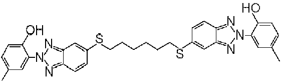 | OH | H | H | Me | H | H | 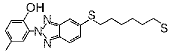 | H | H | 365 | 23900 | 339 |
| Working example 36 | Compound 24 | 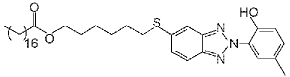 | OH | H | H | Me | H | H | C17H35COOC6H12S | H | H | 365 | 24990 | 357 |
| Comparative example 1 | Compound 22 | 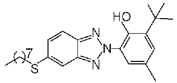 | OH | tBu | H | Me | H | H | C8H17S | H | H | 368 | 24930 | 278 |
| Comparative example 2 | Compound 23 | 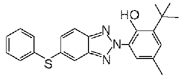 | OH | tBu | H | Me | H | H | PheS | H | H | 367 | 24410 | 279 |

Absolute values of slopes were calculated based on the measurement results of the UV-Vis absorption spectra, where each absolute value is a slope value of a straight line (FIG. 1) connecting the maximum absorption wavelength (Wavelength 1) to the point (Wavelength 2) at which the absorption peak spectrum on the long-wavelength side reaches a light absorbance of 0.1, and the results are as shown in Table 2.

The results for Working Examples 17 to 32 and 37 (the compounds 1 to 11, 13, 15, 17, 18, 21, and 24) each indicated a slope absolute value of 0.060 or higher, which was greater than the slope values of the Comparative Examples 3 and 4 (the compounds 22 and 23).

It was suggested from these results that an ultraviolet absorber represented by the above formula (1), wherein any one of R¹ and R³ to R⁹ is a monovalent sulfur-containing group having a sulfide bond, results in a larger slope value on the long-wavelength side of the light absorption peak when R² is a hydrogen atom compared to a compound in which R² contains a substituent group.

Of these ultraviolet absorbers according to the present invention, it was indicated that compounds whose monovalent sulfur-containing groups each having a sulfide bond are represented by the formula (2) and whose R¹²s are saturated aliphatic hydrocarbon groups (the compounds 1 to 6); compounds whose R¹²s are aromatic ring-containing saturated aliphatic hydrocarbon groups (Compounds 10 and 11); and compounds each containing an oxygen molecule in R¹² (Compounds 13, 15, 17, and 18) all achieved large slopes greater than or equal to 0.065, thus demonstrating their utility.

Among the compounds where R¹² is a saturated aliphatic hydrocarbon group, comparing the compounds 1 and 4 to 6 from each other, they exhibited slope values which were greater in the order of the compound 1 (having 8 carbon atoms; slope value 0.070), the compound 4 (having 12 carbon atoms; slope value of 0.069), the compound 5 (having 16 carbon atoms; slope value of 0.067), and the compound 6 (having 18 carbon atoms; slope value of 0.066), demonstrating a trend where compounds having fewer carbon atoms tend to have larger slope values.

Comparing the compounds 10 (slope value of 0.069) and 11 (slope value of 0.065) with the compound 7 (slope value of 0.061), it was indicated that a compound having R¹² of an aromatic ring-containing saturated aliphatic hydrocarbon group exhibits a larger slope value than a compound having R¹² of an aromatic hydrocarbon group.

**[Table 2]**

| | | Wavelength 1 | | Wavelength 2 | | Absolute value of slope |
|---|---|---|---|---|---|---|
| | | Wavelength (mn) | Absorbance | Wavelength (nm) | Absorbance | |
| Working example 17 | Compound 1 | 366 | 2.7 | 403 | 0.1 | 0.070 |
| Working example 18 | Compound 2 | 365 | 2.6 | 402 | 0.1 | 0.068 |
| Working example 19 | Compound 3 | 361 | 2.6 | 398 | 0.1 | 0.069 |
| Working example 20 | Compound 4 | 365 | 2.7 | 403 | 0.1 | 0.069 |
| Working example 21 | Compound 5 | 365 | 2.6 | 403 | 0.1 | 0.067 |
| Working example 22 | Compound 6 | 365 | 2.6 | 403 | 0.1 | 0.066 |
| Working example 23 | Compound 7 | 363 | 2.6 | 404 | 0.1 | 0.061 |
| Working example 24 | Compound 8 | 363 | 2.5 | 403 | 0.1 | 0.062 |
| Working example 25 | Compound 9 | 360 | 2.6 | 399 | 0.1 | 0.064 |
| Working example 26 | Compound 10 | 366 | 2.8 | 406 | 0.1 | 0.068 |
| Working example 27 | Compound 11 | 365 | 2.7 | 405 | 0.1 | 0.065 |
| Working example 28 | Compound 13 | 364 | 2.5 | 401 | 0.1 | 0.065 |
| Working example 29 | Compound 15 | 365 | 2.6 | 401 | 0.1 | 0.071 |
| Working example 30 | Compound 17 | 363 | 2.8 | 401 | 0.1 | 0.072 |
| Working example 31 | Compound 18 | 365 | 2.5 | 402 | 0.1 | 0.065 |
| Working example 32 | Compound 21 | 365 | 2.4 | 402 | 0.1 | 0.062 |
| Working example 37 | Compound 24 | 365 | 2.5 | 402 | 0.1 | 0.065 |
| Comparative example 3 | Compound 22 | 368 | 2.5 | 409 | 0.1 | 0.059 |
| Comparative example 4 | Compound 23 | 367 | 2.4 | 410 | 0.1 | 0.054 |

### 3. UV-Vis light transmission spectrum

An ultraviolet-visual-infrared spectrophotometer (UH4150V by Hitachi High-Tech Science Corporation) was used to measure an UV-Vis light transmission spectra of the compounds 1 and 22 under the measurement conditions as shown in Table 3 to determine the maximum slopes and wavelengths at which the slopes of the absorption peak spectra on the long-wavelength side reached their maximums.

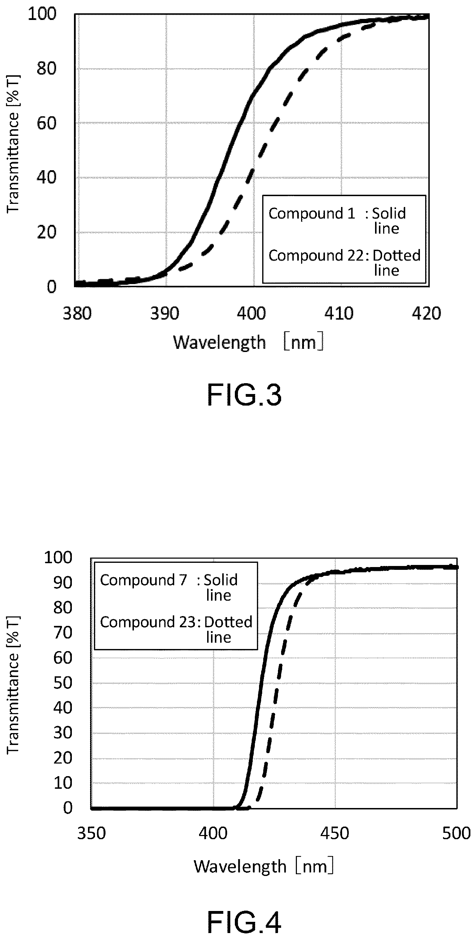

The results are as shown in Table 3 and FIGs. 2 and 3.

Toluene solutions containing the compound 1 (at concentrations of 0.075wt%, 0.75wt%, and 0.4wt%) were subjected to UV-Vis light transmission spectrum measurements. The compound 22 was used as a comparative example, and the measurements were carried out using toluene solutions at concentrations of 0.025wt%, 0.25wt%, and 0.4wt% which were selected to correspond to wavelengths where the transmittances of the compound 1 reached 0%. The results showed that the compound 1 exhibited a larger absolute slope value at each concentration on its transmittance curve. The optical path lengths as measurement conditions were adjusted for each concentration to ensure suitable measurements of the transmission spectra.

In FIGs. 2 and 3, the solid lines indicate the compound 1 while the dotted lines indicate the compound 22. FIG. 2 illustrates light transmission spectra of solutions of the compound 1 at 0.075wt% and 0.75wt% and of solutions of the compound 22 at 0.025wt% and 0.25wt%. FIG. 3 illustrates light transmission spectra of solutions of the compounds 1 and 22 at 0.4wt%.

The slopes on the light transmittance curves were determined as follows. The slope value at, for example, 400 nm was determined as a differential value in transmittance (Δ%T/1 nm) between 0.5 nm before and after the point of measurement. The maximum slope values and wavelengths at which the slopes on the respective transmission curves reached their maximums are as shown in Table 3.

For example, the light transmittance curve of the compound 1 of 0.4wt% exhibited the maximum slope of 9.2 while the one of the compound 22 of 0.4wt% exhibited the maximum slope of 7.7.

In view of the above, the ultraviolet absorber of the present invention exhibited a large slope on the light transmittance curve and had a steep slope (large absolute value of slope) on the longer-wavelength side of the absorption peak, thus suggesting efficient absorption of light from a long-wavelength ultraviolet light region (350 to 400 nm) to a short-wavelength visible light region (400 to 420 nm) while inhibiting the absorption of visible light (400 to 800 nm) by adjusting the added amount thereof.

**[Table 3]**

| | | Measuring conditions | | Absorption peak | |
|---|---|---|---|---|---|
| | | Sample concentration (wt%) | Optical path length | Wavelength at which the slope reached maximum (nm) | Maximum value of slope |
| Working example 33 | Compound 1 | 0.075 | 1 cm | 406 | 9.8 |
| Comparative example 5 | Compound 22 | 0.025 | 1 cm | 408 | 8.8 |
| Working example 34 | Compound 1 | 0.75 | 1 cm | 416 | 9.5 |
| Comparative example 6 | Compound 22 | 0.25 | 1 cm | 406 | 8.4 |
| Working example 35 | Compound 1 | 0.4 | 100 µm | 396 | 9.2 |
| Comparative example 7 | Compound 22 | 0.4 | 100 µm | 400 | 7.7 |

### 4. Resin composition

The compound 1 was added to various types of resin (Polymethyl methacrylate resin, polycarbonate resin, acrylonitrile-butadiene-styrene resin, polyethylene terephthalate resin, cycloolefin polymer resin, and acrylic melamine resin) to prepare resin compositions, which were visually observed to determine if yellowing was inhibited compared to a blank sample.

### Polymethyl methacrylate resin

Here, (0.001 g of) the compound 1, (0.099 g of) polymethyl methacrylate, and (12 g of) chloroform were uniformly mixed, followed by applying about 1 mL of the mixture onto a glass substrate via spin coating under a condition(s) of 1,500 rpm, 20 sec, and then removing the solvent by leaving the glass substate in an oven of 45°C for two hours, thereby preparing a polymethyl methacrylate film having a film thickness of 10 to 50 µm and containing the compound 1 by an amount of 1 wt%.

Further, as a blank sample, (0.1 g of) polymethyl methacrylate and (12 g of) chloroform were uniformly mixed, followed by carrying out operations similar to those described above, thereby preparing a polymethyl methacrylate film having a film thickness of 10 to 50 µm.

### Polycarbonate resin

Here, (0.001 g of) the compound 1, (0.099 g of) a polycarbonate resin and (4 g of) chloroform were uniformly mixed, followed by condensing the chloroform, and then applying 25 µL of the mixture onto a glass slide. Later, the glass slide was left in an oven of 45°C for two hours to remove the solvent, thereby preparing a polycarbonate film having a film thickness of 10 to 50 µm and containing the compound 1 by an amount of 1wt%.

Further, as a blank sample, (0.1 g of) the polycarbonate resin and (4 g of) chloroform were uniformly mixed, followed by carrying out operations similar to those described above, thereby preparing a polycarbonate film having a film thickness of 10 to 50 µm.

### (Resin of acrylonitrile-butadiene-styrene (ABS))

Here, (0.001 g of) the compound 1, (0.099 g of) an ABS resin, and (20 g of) chloroform were uniformly mixed, followed by condensing the chloroform, and then delivering 25 µL of the mixture onto a glass slide by drops. Later, the glass slide was left in an oven of 45°C for two hours to remove the solvent, thereby preparing an ABS film having a film thickness of 10 to 50 µm and containing the compound 1 by an amount of 1 wt%.

Further, as a blank sample, (0.1 g of) an ABS resin and (20 g of) chloroform were uniformly mixed, followed by carrying out operations similar to those described above, thereby preparing an ABS film having a film thickness of 10 to 50 µm.

### Resin of polyethylene terephthalate (PET)

Here, (0.0004 g of) the compound 1 and (0.0396 g of) PET chips were kneaded at 280°C, followed by applying the kneaded product to a glass slide substrate, and then having it air-cooled, thereby preparing a PET film having a film thickness of 20 to 200 µm and containing the compound 1 by an amount of 1wt%.

Further, as a blank sample, 0.045 g of PET was dissolved, followed by carrying out operations similar to those described above, thereby preparing a PET film having a film thickness of 20 to 200 µm.

### Resin of cycloolefin polymer (COP)

Here, (0.001 g of) the compound 1 and (0.099 g of) a COP resin were kneaded at 280°C, followed by applying the kneaded product onto a glass slide substrate, and then having it air-cooled, thereby preparing a COP film having a film thickness of 20 to 200 µm and containing the compound 1 by an amount of 1wt%.

Further, as a blank sample, (0.045 g of) a COP resin was melted, followed by carrying out operations similar to those described above, thereby preparing a COP film having a film thickness of 20 to 200 µm.

### Acrylic melamine resin

Here, (0.0045 g of) the compound 1 was dissolved in 1 mL of THF, followed by uniformly mixing 0.1 mL of the solution with 0.1 mL of an acrylic melamine monomer (bake-drying type topcoat (acrylic melamine): ACRYCITE UB-63 CLEAR by Saito Paint Co., Ltd.), and then applying 0.2 mL of the mixture onto a 1.5×1.5 cm glass slide. This glass slide was then put into an oven and heated at 150°C for two hours to thereby produce an acrylic melamine resin film having a film thickness of 100 to 150 µm and containing the sample by an amount of 1wt%.

Further, as a blank sample, (0.1 mL of) an acrylic melamine monomer and (0.1 mL of) THF were uniformly mixed, followed by carrying out operations similar to those described above, thereby obtaining an acrylic melamine resin film having a film thickness of 100 to 150 µm.

Compositions 7 and 23 were used to prepare acrylic melamine resin compositions following the processes same as those previously described. The resulting acrylic melamine resin films, each having a thickness of 100 to 150 µm, underwent transmittance measurements (FIG. 4).

Comparing the compound 7 to the compound 23, the compound 7 has a smaller molecular weight and therefore results in a higher added amount in terms of molar ratio when they were added in equal weight. Despite this, it was demonstrated that the compound 7 efficiently absorbed light from a long-wavelength ultraviolet light region (350 to 400 nm) to a short-wavelength visible light region (400 to 420 nm) while inhibiting the absorption of visible light (400 to 800 nm), thus showing absorption of light ranging from a long-wavelength ultraviolet light to a short-wavelength visible light while inhibiting yellowing.

It was suggested in view of the above that the ultraviolet absorber according to the present invention exhibits a light absorption peak having a steep slope to the long-wavelength side of the peak (the absolute value of the slope is large), and the ultraviolet absorber efficiently absorbs light from a long-wavelength ultraviolet light region (350 to 400 nm) to a short-wavelength visible light region (400 to 420 nm) while inhibiting the absorption of visible light (400 to 800 nm) by adjusting the added amount thereof; and that the transparent member or resin composition to which the ultraviolet absorber of the present invention is added can efficiently absorb light from a long-wavelength ultraviolet light region (350 to 400 nm) to a short-wavelength visible light region (400 to 420 nm) while inhibiting the light absorption of the visible light region (400 to 800 nm) by adjusting the added amount of the ultraviolet absorber of the present invention, thus capable of inhibiting the yellowing thereof.

## Claims

1. An ultraviolet absorber represented by a formula (1) defined by: 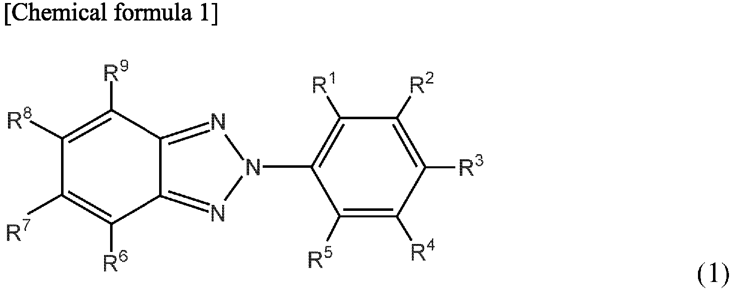 wherein, in the formula, each of R¹ and R³ to R⁹ independently represents a monovalent group selected from a hydrogen atom, a hydrocarbon-containing group, a sulfur-containing group, a nitrogen-containing group, and an oxygen-containing group, at least one of R⁶ to R⁹ represents a monovalent sulfur-containing group that has a sulfide bond, and R² represents a hydrogen atom.

2. The ultraviolet absorber according to claim 1, wherein the monovalent sulfur-containing group that has a sulfide bond is represented by a formula (2) defined by:
[Chemical formula 2]
-(R¹⁰)₁-S-(R¹¹-S)ₘ-R¹² (2)
wherein, in the formula, each of R¹⁰ and R¹¹ independently represents a divalent hydrocarbon-containing group, R¹² is a monovalent hydrocarbon-containing group, 1 is an integer of 0 or 1, and m is an integer of 0 to 3.

3. The ultraviolet absorber according to claim 2, wherein R¹² in the formula (2) is a hydrocarbon group.

4. The ultraviolet absorber according to claim 3, wherein the hydrocarbon group is a saturated aliphatic hydrocarbon group, an aromatic ring-containing aliphatic hydrocarbon group, an aromatic hydrocarbon group, an aliphatic ring-containing aliphatic hydrocarbon group, or an aliphatic cyclic hydrocarbon.

5. The ultraviolet absorber according to claim 2, wherein R¹² in the formula (2) is a hydrocarbon-containing group having an oxygen-containing group.

6. The ultraviolet absorber according to claim 2, wherein R¹² in the formula (2) is a group represented by -A-S-R¹³, wherein A is a divalent hydrocarbon-containing group and R¹³ is a benzotriazole-containing group.

7. A transparent member comprising the ultraviolet absorber according to any one of claims 1 to 6.

8. A resin composition comprising the ultraviolet absorber according to any one of claims 1 to 6 and a resin.
